# EUROPEAN PATENT SPECIFICATION

(11) **EP 3 122 261 B1**
(45) Date of publication and mention of the grant of the patent: **16.11.2022**
(21) Application number: 14887035.5
(22) Date of filing: 26.03.2014
(51) Int. Cl.: A61B 17/115, A61B 17/072, A61B 90/00

(54) **SURGICAL STAPLING APPARATUS**
CHIRURGISCHE KLAMMERVORRICHTUNG
APPAREIL D'AGRAFAGE CHIRURGICAL

(43) Date of publication of application: 01.02.2017
(73) Proprietor: Covidien LP, Mansfield, MA 02048 (US)
(72) Inventor: CHEN, Lin, Shanghai 201101 (CN); ZHAN, Hui, Pudong Shanghai 201203 (CN); ZHANG, Jiangfeng, Shanghai 200092 (CN); HU, Wei, Shanghai 200336 (CN); ZHANG, Xiliang, Shanghai 201114 (CN); WANG, Feng, Shanghai 201114 (CN)
(74) Representative: Soames, Candida Jane
(86) International application number: PCT/CN2014/074065
(87) International publication number: WO 2015/143631

(56) References cited:
- EP-A2- 2 100 561
- EP-A2- 2 163 211
- WO-A1-2014/139440
- WO-A2-2005/037084
- CN-A- 102 440 816
- CN-A- 102 755 180
- CN-A- 103 096 817
- CN-A- 103 371 861

## Description

### BACKGROUND

### Technical Field

The present disclosure relates generally to a surgical stapling apparatus for applying surgical staples to body tissue and, more particularly, to a surgical stapling apparatus for performing circular anastomosis of hollow tissue structures.

### Background of Related Art

Anastomosis refers to the surgical joining of separate hollow tissue sections. Typically, an anastomosis procedure follows surgery in which a diseased or defective section of a hollow tissue structure is removed, thus requiring the joining of the remaining sections of the tissue structure. Depending on the particular procedure being performed and/or other factors, the sections of the tissue may be joined by circular anastomosis, e.g., end-to-end anastomosis, end-to-side anastomosis, or side-to-side anastomosis.

In a circular anastomosis procedure, the two sections of a tubular organ are joined using a stapling apparatus that drives a circular array of staples through each of the sections to join the sections to one another in end-to-end, end-to-side, or side-to-side relation. Typically, any tissue within the newly joined hollow tissue structure is simultaneously cored to clear the passage defined by the hollow tissue structure.

A typical circular anastomosis apparatus includes an elongated shaft having a handle portion at a proximal end and a staple holding component at a distal end. An anvil assembly including an anvil rod and an attached anvil head is mounted to the distal end of the elongated shaft adjacent the staple holding component. In use, the two sections of the tubular organ to be joined are clamped between the anvil head and the staple holding component. The clamped sections are then joined to one another by driving one or more staples from the staple holding component, through the tissue, and into the anvil head to form the staples about the tissue. Examples of such circular anastomosis apparatuses are described in U.S. Patent Nos. 7,857,187 to Milliman ("the Milliman '187 patent") and 6,945,444 to Gresham et al. ("the Gresham '444 patent").

Depending on the type, thickness, and/or other properties of the tissue structures to be joined, it may be desirable to provide a different "minimum tissue gap," wherein the "minimum tissue gap" is defined as the distance between the anvil head and the staple holding component when the stapling apparatus is fully approximated. A need therefore exists for a tissue gap adjustment mechanism that facilitates adjustment of the minimum tissue gap between a plurality of tissue gap settings in a quick and efficient manner. EP 2 163 211 A2 discloses one example of a tissue gap adjustment mechanism.

### SUMMARY

The invention is as defined in the appended claims.

### BRIEF DESCRIPTION OF THE DRAWINGS

Various aspects of the presently disclosed surgical stapling apparatus are described herein with reference to the drawings wherein:
FIG. 1 is a top, side, perspective view from the distal end of the presently disclosed surgical stapling apparatus;
FIG. 2 is a side, perspective view of the handle portion of the surgical stapling apparatus of FIG. 1 wherein one of the handle sections has been removed to shown the internal components of the handle portion;
FIG. 3 is an exploded, perspective view of the surgical stapling apparatus of FIG. 1;
FIG. 3A is a top view of the proximal end of the surgical stapling apparatus of FIG. 1;
FIG. 3B is transverse, cross-sectional view taken along section line 3B-3B of FIG. 3A;
FIG. 3C is transverse, cross-sectional view taken along section line 3C-3C of FIG. 3A;
FIG. 3D is a side, perspective view of the proximal end of the surgical stapling apparatus of FIG. 1 illustrating disengagement of the bushing and the collar from the handle portion;
FIG. 3E is an enlarged, cross-sectional view of the proximal end of the surgical stapling apparatus of FIG. 1 illustrating the initial separation of the handle sections of the handle portion from a closed position towards an open position;
FIG. 3F is an enlarged, perspective view of the handle portion of the surgical stapling apparatus of FIG. 1 with the handle sections of the handle portion disposed in an open position;
FIG. 4 is an enlarged view of the area of detail indicated as "4" in FIG. 1;
FIG. 5 is an exploded, perspective view of the approximation assembly of the surgical stapling apparatus of FIG. 1;
FIG. 5A is a side perspective view of an embodiment of the set screw of the tissue gap adjustment mechanism;
FIG. 6 is a side, perspective view of the proximal end of the handle portion of the surgical stapling apparatus of FIG. 1 with the handle sections removed and the proximal portions of the firing assembly and approximation assembly illustrated;
FIG. 7 is an enlarged view of the area of detail indicated as "7" in FIG. 6;
FIG. 8 is a side, perspective view of the approximation assembly of the surgical stapling apparatus of FIG. 1;
FIG. 9 is an enlarged view if the area of detail indicated as "9" in FIG. 8;
FIG. 9A is an exploded, perspective view of the tissue gap adjustment mechanism of the surgical stapling apparatus of FIG. 1;
FIG. 9B is an enlarged, top view of the adjustment washer of the tissue gap adjustment mechanism of FIG. 9A;
FIG. 10 is a perspective view of the staple pusher assembly of the surgical stapling apparatus of FIG. 1;
FIG. 11 is an exploded, perspective view of the staple pusher assembly of FIG. 10;
FIG. 11A is an enlarged, cross-sectional view of the proximal end of the firing assembly with the trigger lock disposed in a locked position;
FIG. 11B is an enlarged, cross-sectional view of the proximal end of the firing assembly with the trigger lock disposed in an unlocked position;
FIG. 12 is a side, perspective view from the distal end of the elongated body portion of the surgical stapling apparatus of FIG. 1;
FIG. 13 is an enlarged, perspective view of the proximal end of the elongated body portion of FIG. 12;
FIG. 14 is a perspective view from the distal end of the distal bushing of the elongated body portion of FIG. 12;
FIG. 15 is an exploded, perspective view of the elongated body portion of FIG. 12;
FIG. 16 is a perspective view from the proximal end of the proximal bushing of the elongated body portion of FIG. 12;
FIG. 17 is a perspective view of the distal end of the surgical stapling apparatus of FIG. 1 including a safety cap disposed about the distal end of the replaceable stapling assembly of the surgical stapling apparatus of FIG. 1;
FIG. 18 is a perspective of the distal end of the surgical stapling apparatus of FIG. 1 including the safety cap removed from the distal end of the replaceable stapling assembly;
FIG. 19 is a perspective view from the proximal end of the replaceable stapling assembly of the surgical stapling apparatus of FIG. 1;
FIG. 20 is a perspective view from the distal end of the replaceable stapling assembly of the surgical stapling apparatus of FIG. 1;
FIG. 21 is an exploded, perspective view of the replaceable stapling assembly of FIGS. 19 and 20;
FIG. 22 is a longitudinal, cross-sectional view taken along section line 22-22 of FIG. 1;
FIG. 23 is an enlarged view of the area of detail indicated as "23" in FIG. 22; and
FIG. 24 is an enlarged view of the area of detail indicated as "24" in FIG. 22.

### DETAILED DESCRIPTION

Aspects of the presently disclosed surgical stapling apparatus will now be described in detail with reference to the drawings in which like reference numerals designate identical or corresponding elements in each of the several views. Throughout this description, the term "proximal" will refer to the portion of the apparatus closest to the user and the term "distal" will refer to the portion of the apparatus farthest from the user.

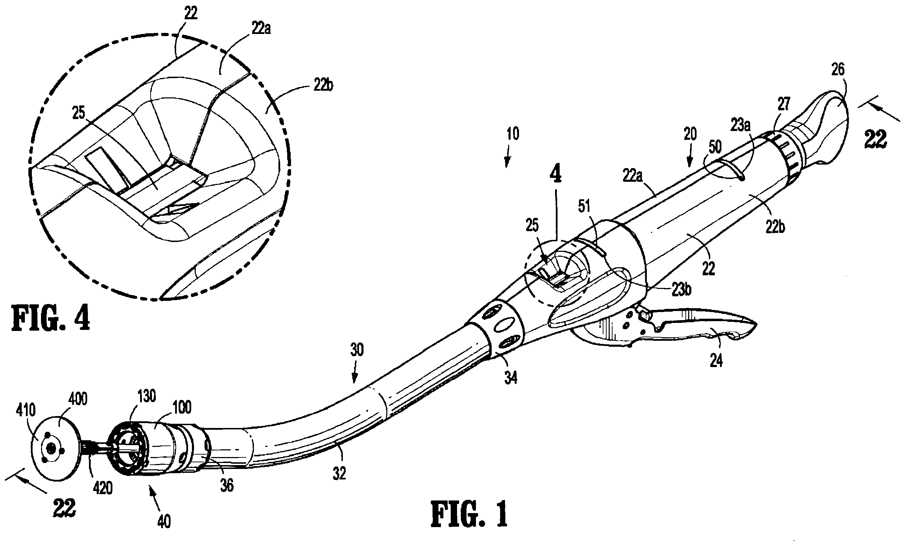

FIGS. 1-24 illustrate an embodiment of the presently disclosed surgical stapling apparatus designated generally by reference numeral 10. Surgical stapling apparatus 10 includes a proximal handle portion 20, an elongated central body portion 30, and a distal head portion 40. Alternatively, it may be desirable to have a substantially straight, shortened central body portion in some surgical procedures, e.g., the treatment of hemorrhoids. The length, shape and/or the diameter of any of the proximal handle portion 20, the central body portion 30, and the distal head portion 40 may also be selected to suit a particular surgical purpose or procedure. Surgical stapling apparatus 10 further includes an anvil assembly 400 coupled at the distal end of distal head portion 40. Anvil assembly 400 includes an anvil head assembly 410 and an anvil center rod assembly 420. Although not described in detail herein, anvil assembly 400 may be configured to include any or all of the features of the anvil assemblies described in the Milliman '187 patent or the Gresham '444 patent.

The various components of surgical stapling apparatus 10 described hereinbelow are configured to facilitate the assembly and disassembly of surgical stapling apparatus 10, thus facilitating the disposal and replacement of those components that are disposable and the sterilization and reassembly of those components that are reusable. The materials used to form the various components of surgical stapling apparatus 10 will depend upon the strength requirements of the particular component and the use requirements of the particular component, e.g., whether the component is reusable or disposable. The reusable components, for example, may generally be formed from thermoplastics including polycarbonates, and metals including stainless steel and aluminum, that are suited to withstand repeated sterilization procedures, e.g., autoclaving.

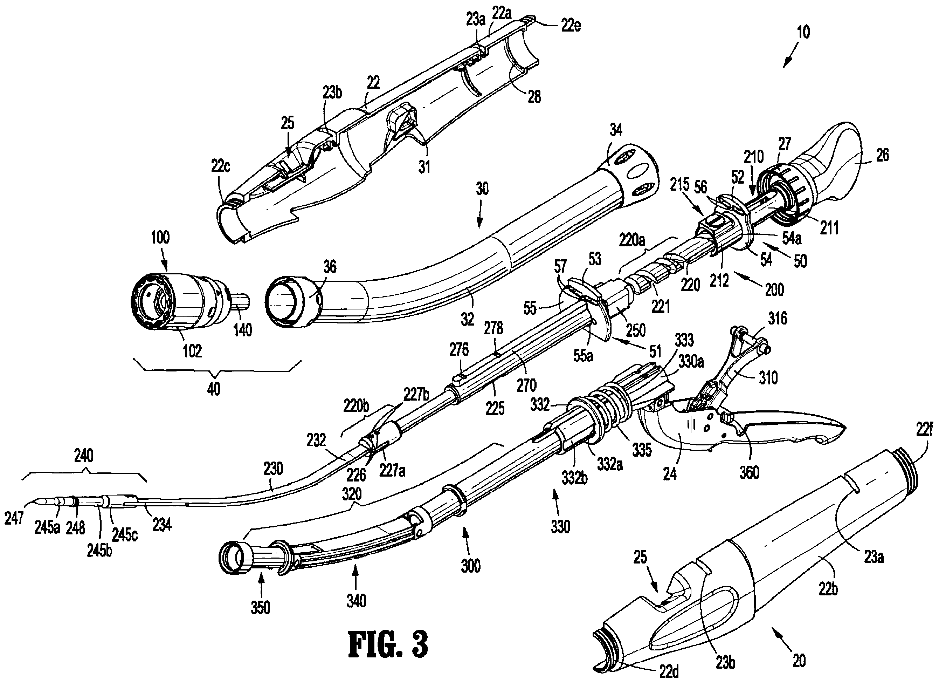

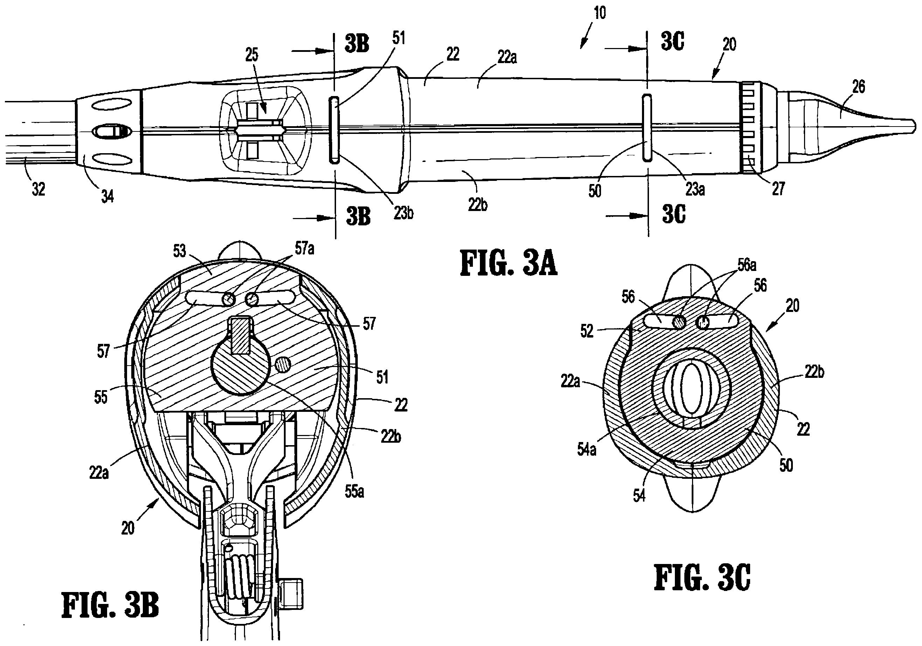

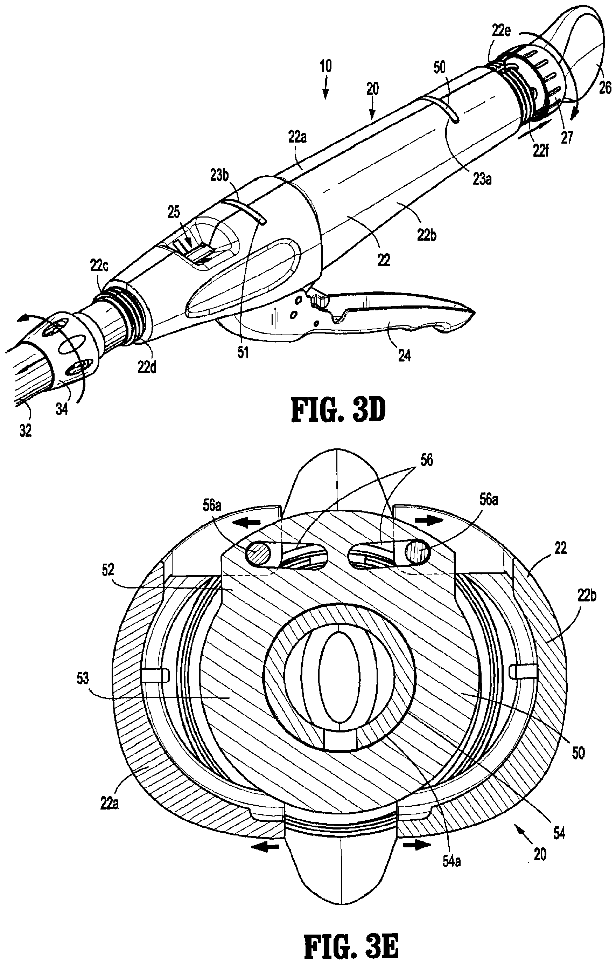

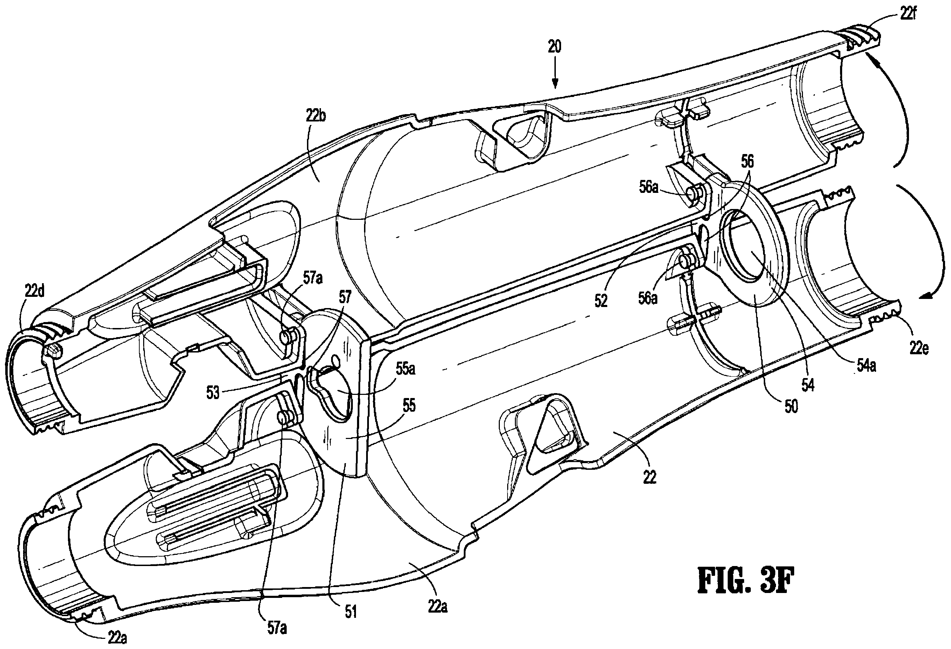

Referring to FIGS. 1-3, proximal handle portion 20 of surgical stapling apparatus 10 includes a stationary handle 22, a firing trigger 24, and a rotatable approximation knob 26. Stationary handle 22 is formed from first and second releasably engagable handle sections 22a, 22b (FIG. 3) that cooperate to house and support the internal components of handle portion 20, e.g., the proximal components of an approximation assembly 200 (FIG. 3) and a firing assembly 300 (FIG. 3). Proximal handle portion 20 and the internal components thereof will be described in greater detail below.

As mentioned above, stationary handle 22 is formed from first and second handle sections 22a, 22b that cooperate to house and support the internal components of handle portion 20. Alternatively, stationary handle 22 may be unitarily formed or formed from multiple handle sections. Handle sections 22a, 22b can be configured as reusable, sterilizable components, or, alternatively, can be configured as disposable components.

Referring specifically to FIG. 3, each handle section 22a, 22b includes a threaded distal extension 22c, 22d. Distal extensions 22c, 22d cooperate to define a generally annular threaded member for releasably engaging proximal bushing 34 of central body portion 30. Engagement between distal extensions 22c, 22d and proximal bushing 34 releasably secures outer tube 32 and handle portion 20 to one another and also secures handle sections 22a, 22b to one another at the distal ends thereof. As an alternative to threaded engagement, proximal bushing 34 may be releasably engaged about distal extensions 22c, 22d of handle sections 22a, 22b via any other suitable mechanism including friction-fitting, snap-fitting, luer-locking, inter-fitting, etc. Handle sections 22a, 22b further include threaded proximal extensions 22e, 22f, respectively, that cooperate to define a generally annular threaded member for releasably engaging collar 27 of approximation knob 26. Similarly as above, engagement between proximal extensions 22e, 22f and collar 27 rotatably secures approximation knob 26 and handle portion 20 to one another and also secures handle sections 22a, 22b to one another at the proximal ends thereof. Collar 27 is rotatably secured to approximation knob 26. Alternatively, collar 27 can be formed separately from approximation knob 26.

Referring also to FIGS. 3A-3C, handle sections 22a, 22b are pivotably coupled to one another via a pair of support members, e.g., support discs 50, 51, and a plurality of pin-slots engagements. Support discs 50, 51 each define an upper engagement portion 52, 53 and a lower generally annular portion 54, 55 defining a respective aperture 54a, 55a. Upper engagement portions 52, 53 of support discs 50, 51 are disposed within respective slots 23a, 23b (FIG. 3A) defined by cooperating slot portions of handle sections 22a, 22b. A pair of pins 56a, 57a extend through respective apertures defined within handle sections 22a, 22b on opposite sides of each of slots 23a, 23b and through slots 56, 57 defined through upper engagement portions 52, 53 of support discs 50, 51 to pivotally secure handle sections 22a, 22b to support discs 50, 51 and to one another. Slots 56, 57 are dimensioned to permit lateral translation of pins 56a, 57a along slots 56, 57 and relative to one another as well as rotation of pins 56a, 57a within slots 56, 57. Apertures 54a, 55a defined by the lower portions 54, 55 of support discs 50, 51, respectively, are configured to receive rotatable sleeve 210 and indicator bar 270 of approximation assembly 200. The lower portions 54, 55 of support discs 50, 51 are configured to position and support approximation assembly 200 within stationary handle 22. As will be described in detail below with respect to the disassembly of stationary handle 22, the slot-pin engagement of handle sections 22a, 22b and support discs 50, 51 allows for translational and rotational movement of handle sections 22a, 22b relative to one another between a closed position (FIG. 3A), wherein stationary handle 22 encloses the proximal components of approximation assembly 200 and firing assembly 300, and an open position (FIG. 3F), wherein access to approximation assembly 200 and firing assembly 300 is provided to facilitate replacement of any or all of such components.

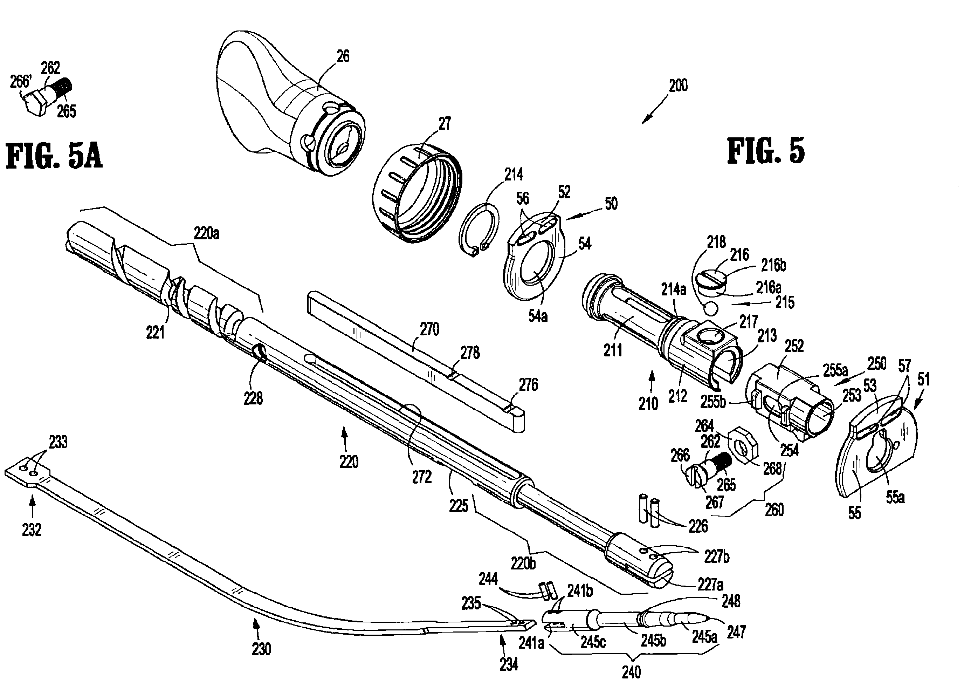

Referring to FIGS. 4 and 5, stationary handle 22 defines an indicator window 25 through which visual indicators 276, 278 disposed on indicator bar 270 may be viewed. Indicator window 25 may be formed via a hole or aperture defined by one or both of handle sections 22a, 22b. Alternatively, indicator window 25 may be formed from a transparent portion of one or both of handle sections 22a, 22b. Visual indicators 276, 278 are longitudinally-spaced along indicator bar 270 and, when visible through indicator window 25, indicate the position of anvil assembly 400 (FIG. 1) in relation to stapling assembly 100, e.g., whether the anvil assembly 400 (FIG. 1) is in a position spaced-apart from stapling assembly 100 (visual indicator 276) or an approximated position in juxtaposed alignment with stapling assembly 100 (visual indicator 278). Approximation assembly 200 is detailed below.

Referring to FIGS. 5-9, approximation assembly 200 of surgical stapling apparatus 10 (FIG. 1) includes an approximation knob 26, a collar 27, a rotatable sleeve 210, a drive screw 220, a screw extension 230, an anvil retainer 240, a screw stop 250, and a tissue gap adjustment mechanism 260.

Rotatable sleeve 210 includes a substantially cylindrical hollow body portion 211 and a distal housing 212 that cooperate to define a central bore 213. A clip 214 is received within an annular groove 214a formed about body portion 211. Support disc 50, as mentioned above, is configured to receive body portion 211 through aperture 54a thereof. Clip 214 and distal housing 212 abut support disc 50 on either side thereof to axially fix sleeve 210 and stationary handle 22 relative to one another while permitting rotation of sleeve 210 in relation to stationary handle 22.

With particular reference to FIG. 5, rotatable sleeve 210 further includes a ball detent assembly 215 having a plug 216 and a detent member. The detent member may include a ball 218. Ball 218 extends into central bore 213 of rotatable sleeve 210 from a recess 217 of distal housing 212 and is received in a helical channel 221 of drive screw 220. Plug 216 includes a body 216a configured for receipt within recess 217 and defines a semi-spherical concavity (not explicitly shown) for receiving a side of ball 218 opposite screw 220, and a head 216b configured for engagement with distal housing 212, e.g., via threaded-engagement, friction-fitting, etc. Once engaged with distal housing 212, plug 216 inhibits ball 218 from backing out of helical channel 221 of screw 220. The recess 217 can be defined only as a spherical recess (not shown) on an inner wall of the distal housing 212 which is configured to receive ball 218. In this case, the plug 216 is not required.

A proximal end of body portion 211 of rotatable sleeve 210 extends through an opening 21 in a proximal end of stationary handle 22. Approximation knob 26 is affixed to the proximal end of body portion 211 of rotatable sleeve 210 such that rotation of knob 26 causes concurrent rotation of rotatable sleeve 210. Approximation knob 26 may be releasably or permanently affixed to rotatable sleeve 210, e.g., via snap-fitting, friction-fitting, an adhesive, welding, and/or mechanical fasteners. Approximation knob 26 and/or the proximal end of body portion 211 of rotatable sleeve 210 may include one or more complementary protrusions and/or slots (not explicitly shown) to rotatably fix approximation knob 26 relative to sleeve 210.

Referring again to FIGS. 5-9, a proximal portion 220a of screw 220 includes helical channel 221 and is dimensioned to be slidably positioned within central bore 213 (FIG. 5) of rotatable sleeve 210. As mentioned above, ball 218 (FIG. 5) of ball detent mechanism 215 extends into helical channel 221 of screw 220. Since sleeve 210 is axially fixed with respect to stationary handle 22, rotation of sleeve 210 about screw 220 causes ball 218 (FIG. 5) to move along channel 221 of screw 220 to effect axial movement of screw 220 within stationary handle 22. Although shown having helical channel 221 configured to receive ball 218 (FIG. 5), it is envisioned that screw 220 may alternatively include a helical thread (not shown) on an outer surface thereof configured to be received within a channel or groove (not shown) formed on an inner surface of sleeve 210. Further, as an alternative to ball detent assembly 215, approximation assembly 200 may include a pin or other suitable mechanism for operably coupling rotatable sleeve 210 and screw 220 to one another.

Distal portion 220b of screw 220 defines a transverse slot 227a and a pair of throughbores 227b formed perpendicular to transverse slot 227a. Transverse slot 227a is configured to receive a proximal end of screw extension 230 and throughbores 227b are configured to receive pins 226 for securing screw extension 230 to screw 220.

Indicator bar 270 is positioned between proximal portion 220a and distal portion 220b of screw 220. Indicator bar 270 is seated within a longitudinal recess 272 defined along screw 220 and may be secured therein in any suitable manner, e.g., via snap-fitting, friction-fitting, an adhesive, welding, and/or mechanical fasteners. As detailed above, indicator bar 270 includes first and second indicators 276, 278 configured to be viewed through indicator window 25 (FIG. 4) to provide an indication that the anvil assembly 400 (FIG. 1) is in the spaced-apart position or the approximated position, respectively. Indicators 276, 278 may be of any suitable color(s), symbol(s) or may include any other suitable feature, e.g., reflective features, a light source (LED), etc., to facilitate the visualization of visual indicators 276, 278 through window 25 (FIG. 4). Other suitable indicator mechanisms are disclosed in the Milliman '187 patent and the Gresham '444 patent.

With continued reference to FIGS. 5-9, screw extension 230 includes a flexible flat band having proximal and distal portions 232, 234. Although shown including only a single flexible flat band, it is envisioned that screw extension 230 may include more than one flexible flat band. Alternately, it is envisioned that screw extension 230 may have other than a flexible flat band configuration. For example, screw extension 230 may be semi-circular or circular in cross-section. The flexibility of screw extension 230 permits movement of screw extension 230 through curved elongated outer tube 32 (FIG. 3). Proximal portion 232 of screw extension 230 includes a pair of holes 233 dimensioned to receive pins 226 for securing proximal portion 232 of screw extension 230 within transverse slot 227a of screw 220. Alternatively, other fastening techniques may be used to secure screw extension 230 to screw 220, e.g., welding, crimping, etc. Distal portion 234 of screw extension 230 is configured to be received within a transverse slot 241a formed in a proximal end 242 of anvil retainer 240 to fasten anvil retainer 240 to distal end 234 of screw extension 230. In the illustrated embodiment, a pair of pins 244 extend through a pair of openings 241b defined in proximal end 242 of anvil retainer 240 and holes 235 in distal portion 234 of screw extension 230 to secure screw extension 230 to anvil retainer 240. Alternately, distal portion 234 of screw extension 230 may be secured within slot 241a using any other fastening technique, e.g., screws, crimping, brazing, welding or the like, suitable for securing distal portion 234 of screw extension 230 to anvil retainer 240.

Anvil retainer 240 includes a trocar portion 245a, a body portion 245b, and an attachment portion 245c. Trocar portion 245a includes a blunt trocar tip 247, although other configurations are also contemplated. Body portion 245b is substantially cylindrical and has a diameter which is larger than the diameter of trocar portion 245a. An annular protrusion 248 is disposed about body portion 245b of anvil retainer 240 and is configured to engage anvil assembly 400 (FIG. 1) to retain anvil assembly 400 (FIG. 1) about anvil retainer 240. Alternatively, protrusion 248 need not be annular or may include different attachment structure, e.g., recesses, grooves, etc.

In use, when approximation knob 26 is manually rotated, rotatable sleeve 210 is likewise rotated about the proximal portion 220a of screw 220. Since sleeve 210 is axially fixed with respect to stationary handle 22, and with ball 218 (FIG. 5) disposed within helical channel 221 of screw 220, axial rotation of sleeve 210 about screw 220 causes ball 218 to move along channel 221 of screw 220 to thereby urge screw 220 to translate axially within stationary handle 22 and relative to sleeve 210. Upon axial translation of screw 220, screw extension 230, which is fastened to the distal end of screw 220, and anvil retainer 240, which is fastened to the distal end of screw extension 230, are moved axially through outer tube 32 of elongated body portion 30. Thus, referring particularly to FIG. 1, with anvil assembly 400 releasably engaged about the distal end of anvil retainer 240, knob 26 may be rotated to effect movement of anvil assembly 400 relative to stapling assembly 100 between an unapproximated position spaced-apart from stapling assembly 100 and an approximated position positioned adjacent to stapling assembly 100.

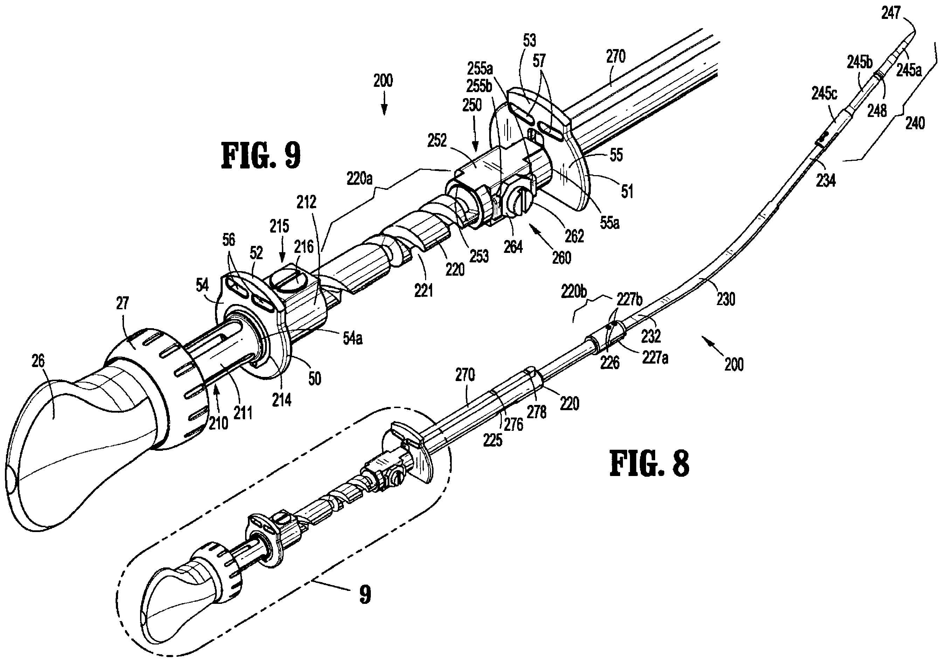

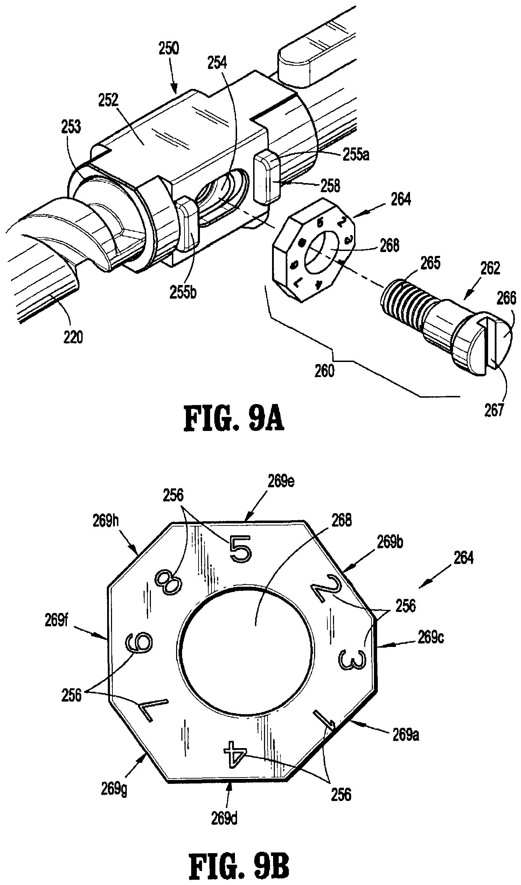

With additional reference to FIGS. 9A-9B, approximation assembly 200 further includes a screw stop 250 disposed about screw 220 and configured to function as a proximal stop for defining the minimum tissue gap between anvil assembly 400 and stapling assembly 100 (see FIG. 1). More specifically, when stapling device 10 is in a fully approximated position, screw stop 250 abuts at stop surface formed at the distal end of distal housing 212 of rotatable sleeve 210, inhibiting further proximal translation of screw 220 within stationary handle 22, thereby defining the minimum tissue gap between anvil assembly 400 and stapling assembly 100 (see FIG. 1). Tissue gap adjustment mechanism 260 adjustably couples screw stop 250 to screw 200 to facilitate adjustment of the minimum tissue gap by adjusting the longitudinal position of screw stop 250 on screw 220. Tissue gap adjustment mechanism 260 is described in detail below.

Tissue gap adjustment mechanism 260, as mentioned above, is configured to selectively adjust the longitudinal position of screw stop 250 on screw 220, thereby enabling discrete adjustment of the minimum tissue gap between anvil assembly 400 and stapling assembly 100 (see FIG. 1). In particular, tissue gap adjustment mechanism 260 may be configured to permit adjustment of the minimum tissue gap through a plurality of pre-determined interval steps, e.g., a step size of about 0.15mm, between about 4.55mm and about 5.45mm, although a greater or lesser range and/or greater or lesser interval step sizes are also contemplated, depending on a particular surgical purpose. Any suitable number of intervals and/or varying step sizes may also be provided.

As best shown in FIG. 9A, tissue gap adjustment mechanism 260 includes a set screw 262 and an asymmetrical polygonal washer 264, e.g., an asymmetrical octagonal washer (although other configurations are also contemplated). Screw stop 250 includes a housing 252 that defines a central bore 253 configured to receive screw 220, and a transverse slot 254 configured to receive set screw 262. First and second spaced-apart flanges 255a, 255b extend transversely from housing 252 on either side of transverse slot 254 for retaining asymmetrical washer 264 therebetween. Screw 220 includes a threaded aperture 228 (FIG. 5) configured to receive threaded shaft 265 of set screw 262 to retain screw stop 250 in a fixed position about screw 220. Head 266 of set screw 262 includes a slot 267 configured to receive a screw driver (not shown) or other suitable tool for driving set screw 262 into or out of threaded aperture 228. As opposed to a slot 267, other suitable tool-engaging features, e.g., recesses and/or protrusions, are also contemplated. For example, as shown in FIG. 5A, the set screw 262 includes a polygonal head, e.g., a pentagonal head, configured to be engaged by a ratchet or wrench. Further, as opposed to set screw 262 received within threaded aperture 228 (FIG. 5) of screw 220, screw 220 may include a post (not shown) or other suitable feature supported thereon for receipt within and longitudinal positioning relative to transverse slot 254 of stop member 250.

Referring to FIGS. 9A-9B, asymmetrical washer 264 defines an aperture 268 and includes a plurality of outer peripheral flats or sides 269a-269h, e.g., eight sides, although greater or fewer sides are also contemplated. Due to the asymmetrical configuration of washer 264, the position of set screw 262 within transverse slot 254 of screw stop 250 is varied depending on which opposed sides 269a-269h of washer 264 are positioned between flanges 255a, 255b. With threaded aperture 228 of screw 220 receiving threaded shaft 265 of set screw 262, varying the position of set screw 262 within transverse slot 254 likewise varies the longitudinal position of screw stop 250 relative to screw 220, thereby varying the size of the minimum tissue gap. For example, as the screw stop 250 is moved proximally on the screw 220, the minimum tissue gap is increased because the screw stop 250 will engage sooner. Sides 269a-269h may include indicators 256 and flange 255a may also include an indicator 258 to allow the user to readily ascertain the setting of tissue gap adjustment mechanism 260. The minimum tissue gap corresponding to each setting of an exemplary configuration of asymmetrical washer 264 is provided in the following table, although other configurations are also contemplated.

| Setting | Minimum Tissue Gap (mm) |
|---|---|
| 1 | 4.55 |
| 2 | 4.70 |
| 3 | 4.85 |
| 4 | 5.00 |
| 5 | 5.00 |
| 6 | 5.15 |
| 7 | 5.30 |
| 8 | 5.45 |

With general reference to FIGS. 5-9B, in order to adjust the minimum tissue gap, stationary handle 22 (FIG. 1) is disassembled (or prior to assembly), set screw 262 is loosened, and asymmetrical washer 264 is rotated about set screw 262 and relative to screw stop 250 to the desired position. Once the desired position is achieved, set screw 262 may be re-tightened to maintain screw stop 250 in the desired position on screw 220, thus defining the desired minimum tissue gap. As an alternative to disassembling stationary handle 22 (FIG. 1), a hole or opening may be provided in stationary handle 22 (FIG. 1) to provide direct access to tissue gap adjustment mechanism 260 without the need to disassemble stationary handle 22 (FIG. 1). Alternatively, the tissue gap adjustment mechanism 260 can be preset to one of the predefined settings by the manufacturer.

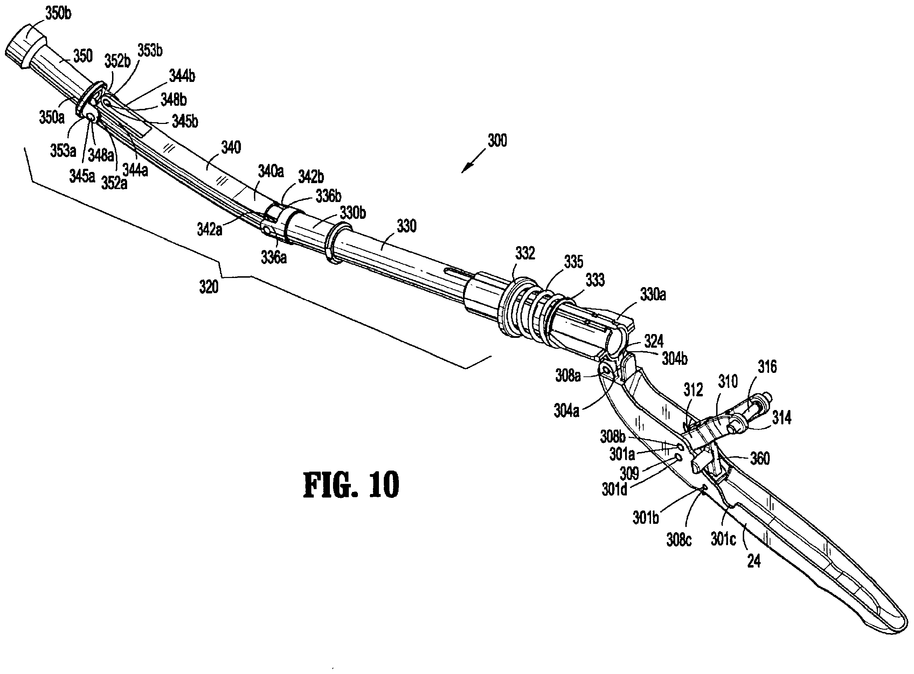

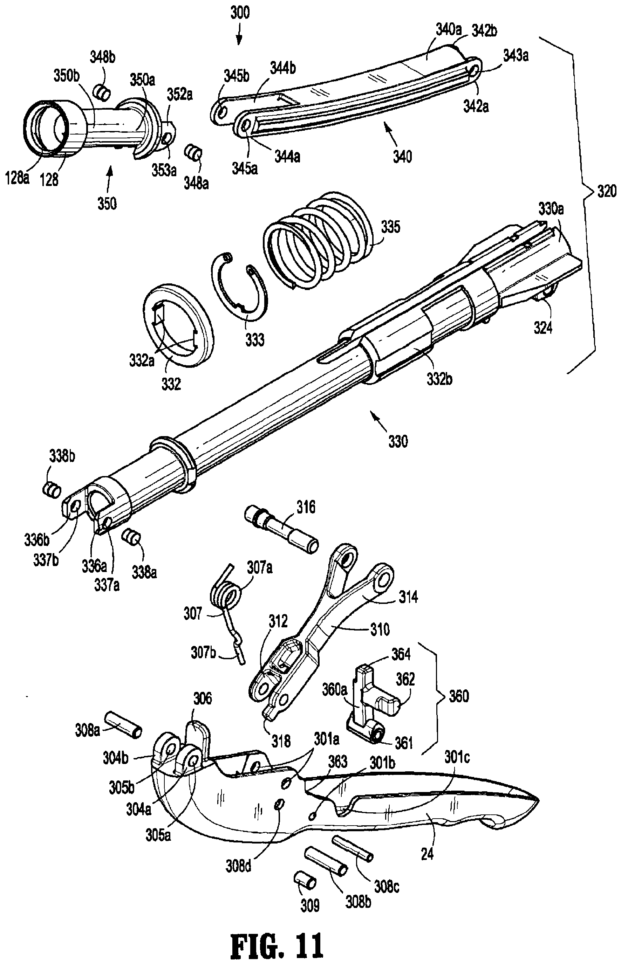

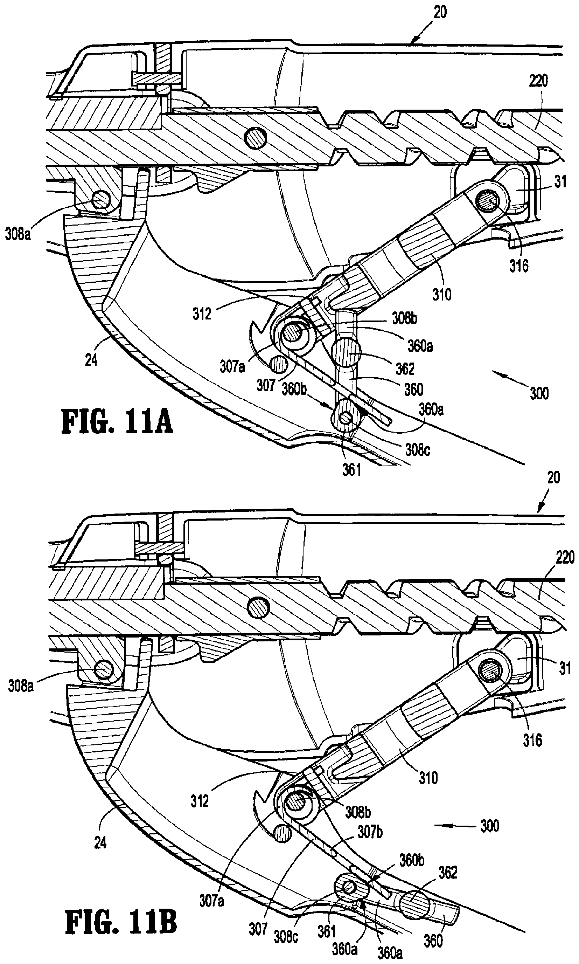

Firing assembly 300 will now be described with reference to FIGS. 10 and 11. Firing assembly 300 includes trigger 24, a firing link 310, and a pusher linkage 320. Pusher linkage 320 includes an elongated pusher tube 330, a pusher link 340, and a pusher end tube 350. Pusher linkage 320 is configured for transferring force from proximal handle portion 20 (FIG. 1) to distal head portion 40 (FIG. 1) to fire stapling assembly 100 (FIG. 1). Although shown as a three-part assembly, it is envisioned that pusher linkage 320 may include one or more additional sections. Optionally, firing assembly 300 includes a trigger lock 360, which will be described in further detail below.

Trigger 24 is configured for operable engagement by a user. Trigger 24 may support a cushioned gripping surface (not shown) formed of neoprene, rubber or the like. The cushioned gripping surface provides a non-slip cushioned surface to make actuation of stapling apparatus 10 (FIG. 1) more comfortable to a surgeon. Alternatively, trigger 24 may be formed of perforated stainless-steel or other metal to facilitate sterilization. The distal end of trigger 24 includes a pair of flanges 304a, 304b each defining an opening 305a, 305b, respectively. Flanges 304a, 304b are configured for pivotal connection with a pair of flanges 324 formed on proximal end 330a of elongated pusher tube 330 of pusher linkage 320 by a pivot member 308a. Alternatively, pusher linkage 320 may include a coupling member (not shown) integrally formed with or fixedly secured to proximal end 330a of pusher tube 330 for pivotally connecting trigger 24 with pusher linkage 320.

Trigger 24 further includes a lockout feature, e.g., protrusion 306, extending from a distal end of trigger 24 adjacent flanges 304a, 304b. Protrusion 306 is configured to engage the distal end of screw 220 (FIG. 5) of approximation assembly 200 (FIG. 5) when approximation assembly 200 (FIG. 5) is in an unapproximated position to prevent accidental actuation of trigger 24 before the anvil assembly (FIG. 1) has been moved to the approximated position. When approximation assembly 200 (FIG. 5) is in the approximated position, recess 225 (FIG. 5) formed in the distal end of screw 220 (FIG. 5) is in alignment with protrusion 306, thereby permitting actuation of trigger 24, i.e., pivotal movement of trigger 24 about pivot member 308a towards stationary handle 22. A biasing member (not shown) may also be provided for biasing trigger 24 towards an unactuated position and for returning trigger 24 to the unactuated position after firing.

Referring still to FIGS. 10 and 11, trigger 24 further includes a first opening 301a, a second opening 301b, a notch 301c, and an indicator member 309. First opening 301a in trigger 24 is configured to receive a pin 308b for pivotally connecting trigger 24 with firing link 310. Second opening 301b in trigger 24 is configured to receive a pin 308c for pivotally connecting trigger 24 with trigger lock 360. Notch 301c is configured to releasably retain protrusion 362 of trigger lock 360 therein to retain trigger lock 360 in an unlocked position. Indicator member 309 is fixedly retained within a third opening 308d and is configured to engage an indicator member 318 of firing link 310 to provide an audible and/or tactile indication to a user as stapling apparatus 10 (FIG. 1) is fired.

Firing link 310 includes a distal end 312 pivotally secured to trigger 24 by a pivot member 308b received through opening 301a. A proximal end 314 of firing link 310 supports a pivot member 316 which is pivotally secured within a slot 31 (FIG. 3) formed on each internal wall of handle sections 22a, 22b (FIG. 3). Alternatively, the pivot member 316 can be formed integrally with the firing link 310. Pivot member 316 is free to move vertically within slots 31 (FIG. 3). Although not shown, it is contemplated that a spring may be supported within handle sections 22a, 22b (FIG. 3) to urge pivot member 316 towards the bottom of slot 31 (FIG. 3), as provided in the Milliman '187 patent. Indicator member 318 is formed on distal end 312 of firing link 310 and is configured to engage indicator member 309 formed on trigger 24 during firing of stapling apparatus 10 (FIG. 1), as mentioned above.

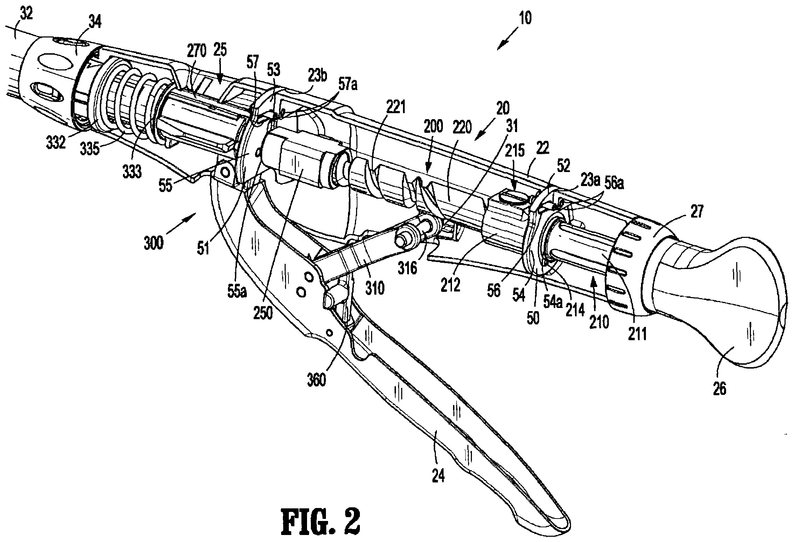

As noted above, pusher linkage 320 includes an elongated pusher tube 330, a pusher link 340 and a pusher end tube 350. A spring 335 received about proximal end 330a of elongated pusher tube 330 is configured to bias pusher linkage 320 proximally to a retracted position. Spring 335 is retained about proximal end 330a of elongated pusher tube 330 via a ring member 332 and a clip 333, although other configurations for retaining spring 225 about proximal end 330a of elongated pusher tube 330 are also contemplated. Ring member 332 is positionable distally of spring 335 and includes a pair of opposed, inwardly-extending protrusions 332a configured for slidable receipt within elongated recesses 332b defined on opposed sides of pusher linkage 320. Clip 333 is configured for engagement about proximal end 330a of elongated pusher tube 330 proximally of spring 335. The ring member 332 sbuts an inner wall of a distal end of stationary handle 22 (FIG. 2) such that spring 335 urges clip 333 and pushes linkage 320 proximally.

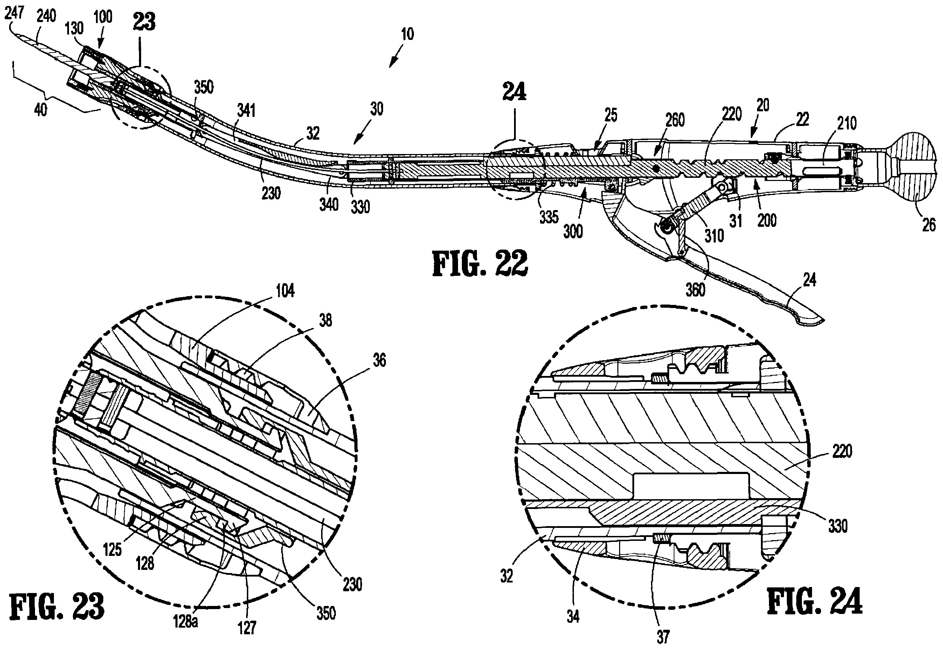

Distal end 330b of pusher tube 330 includes a pair of flanges 336a, 336b each defining an opening 337a, 337b, respectively. Each of openings 337a, 337b is configured to receive a pivot pin 338a, 338b, respectively, to pivotally secure a proximal end 340a of pusher link 340 with distal end 330b of elongated pusher tube 330. Pusher link 340 includes an elongated member defining a channel 341 (FIG. 22) extending substantially the length thereof. As shown, pusher link 340 may be slightly curved along the length thereof. Channel 341 (FIG. 22) is configured to receive screw extension 230 of approximation assembly 200 therethrough (FIG. 5). Proximal end 340a of pusher link 340 includes a first pair of flanges 342a, 342b each defining an opening 343a, (not shown) sized to receive respective pivot pin 338a, 338b for pivotally connecting pusher link 340 and elongated pusher tube 330. A distal end 340b of pusher link 340 includes a second pair of flanges 344a, 344b, each defining an opening 345a, 345b sized to receive a pivot pin 348a, 348b, respectively, for pivotally connecting pusher link 340 and pusher end tube 350.

A proximal end 350a of pusher tube 350 includes a pair of flanges 352a, 352b each defining an opening 353a, 353b configured for receiving respective pivot pins 348a, 348b for pivotally connecting pusher tube 350 with pusher link 340. A distal end 350b of pusher end tube 350 is configured to selectively engage the stapling assembly 100 (FIG. 1), as will be detailed below.

With reference to FIGS. 11A and 11B, as noted above, firing assembly 300 may optionally include trigger lock 360. Trigger lock 360 includes a body 360a and a base 361 defining a throughbore for receiving a pin 308c. Pin 308c is received within the throughbore of base 361 to pivotably couple trigger lock 360 to trigger 24. Base 361 defines a radially asymmetric configuration, e.g., a tear-drop cross-sectional configuration, forming a pair of contact surfaces 361a, 361b on either side of the throughbore that receives pin 308c. Trigger lock 360 further includes a protrusion 362 extending from body 360a of trigger lock 360. Trigger lock 360 is rotatable relative to trigger 24 between an unlocked position, wherein protrusion 362 is received within notch 301c (FIG. 11) of trigger 24, and a locked position, wherein protrusion 362 is received within cut-out 363 (FIG. 11) of trigger 24 and free end 364 of body 360a of trigger lock 360 is disposed in close approximation or abutting relation with firing link 310 to inhibit actuation of trigger 24.

A biasing member 307, e.g., a torsion spring, is disposed about pivot member 308b, which, as mentioned above, is received through opening 301a of trigger 24 and distal end 312 of firing link 310 to pivotally secured firing link 310 to trigger 24. Biasing member 307 includes a coiled portion 307a and a flat portion 307b. Coiled portion 307a is disposed about pivot member 308b, while flat portion 307b extends proximally from coiled portion 307a along trigger 24. Coiled portion 307a biases flat portion 307b towards trigger 24. More specifically, in the locked position of trigger lock 360, as shown in FIG. 11A, flat portion 307b of biasing member 307 is biased into contact with contact surface 361a of base 361 of trigger lock 360 to urge trigger lock 360 in a counter-clockwise direction as viewed in FIG. 11A. In this position, biasing member 307 maintains trigger lock 360 in the locked position and inhibits accidental dislodgment of trigger lock 360 from the locked position. In the unlocked position of trigger lock 360, as shown in FIG. 11B, flat portion 307b of biasing member 307 is biased into contact with contact surface 361b of base 361 of trigger lock 360 to urge trigger lock 360 in a clockwise direction as viewed in FIG. 11B. In this position, biasing member 307 maintains trigger lock 360 in the unlocked position and inhibits accidental dislodgment of trigger lock 360 from the unlocked position. Thus, biasing member 307 establishes a bistable configuration of trigger lock 360, e.g., wherein trigger lock 360 is stable in both the locked position and the unlocked position.

Other suitable trigger locks are described in U.S. Patent 7,303,106 to Milliman et al., and the Milliman '187 patent and the Gresham '444 patent. Firing assembly 300 may further include a feedback mechanism similar to that disclosed in the Milliman '187 patent.

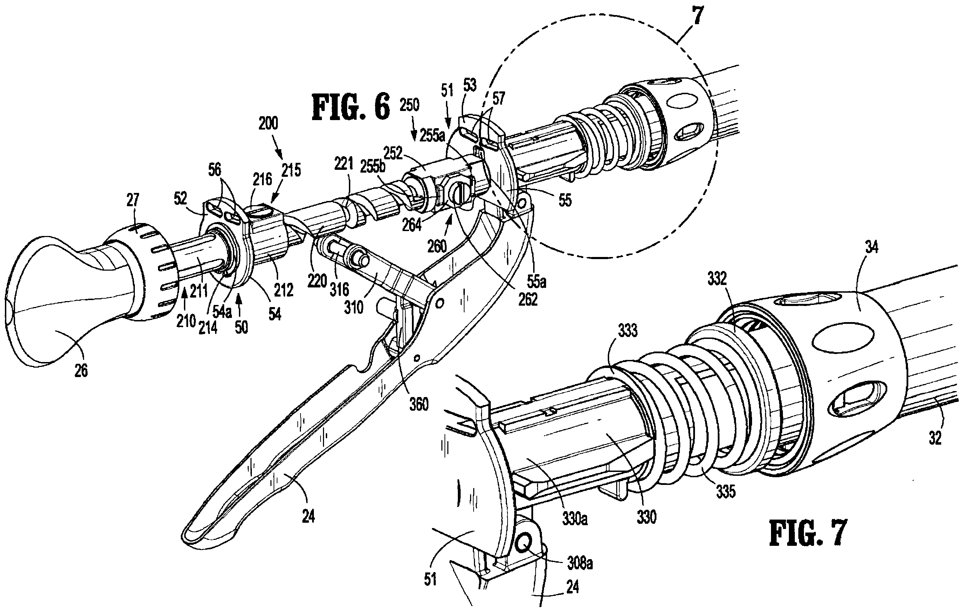

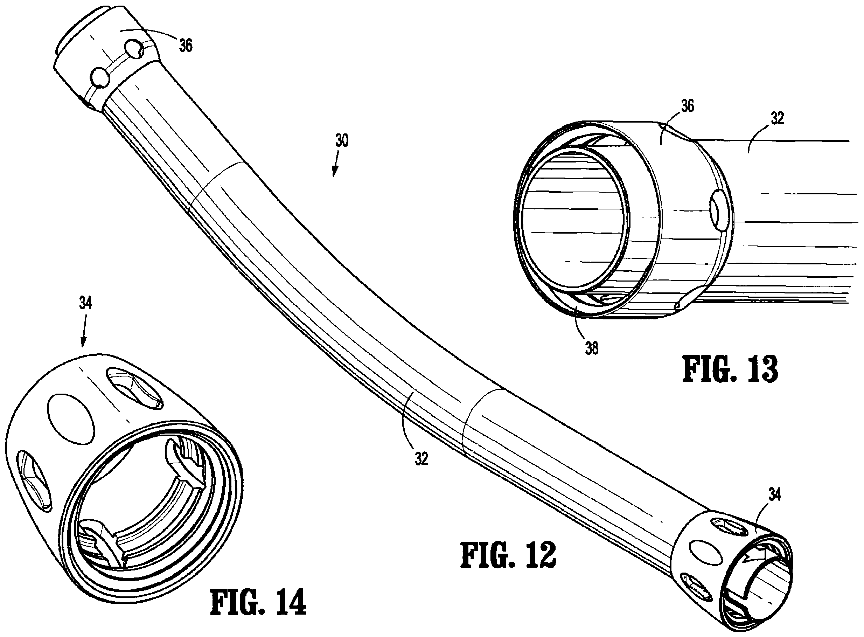

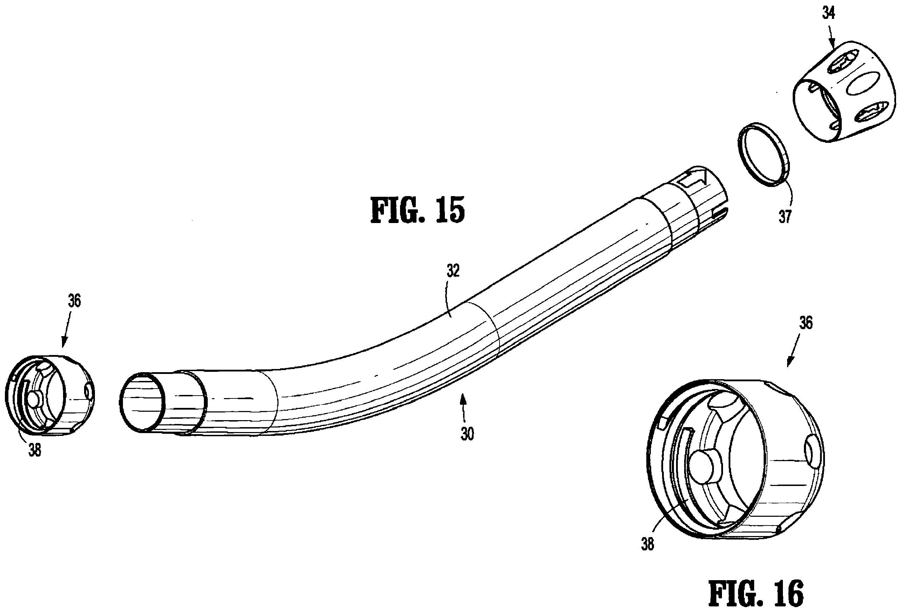

With reference to FIGS. 12-16, elongated central body portion 30 of surgical stapling apparatus 10 (FIG. 1) includes a curved elongated outer tube 32, a proximal bushing 34 (FIG. 16), and a distal bushing 36 (FIG. 14). Outer tube 32 is configured to slidably receive components of approximation assembly 200 (FIG. 3) and firing assembly 300 (FIG. 3). Proximal bushing 34 is rotatably coupled about outer tube 32 via a ring 37 and is configured to enable releasable threaded engagement of the proximal end of outer tube 32 with stationary handle 22 of handle portion 20 (FIG. 6). Distal bushing 36, is engaged about the distal end of outer tube 32, e.g., via friction-fitting, snap-fitting, adhesion, or other suitable engagement, and is configured to enable releasable engagement of replaceable stapling assembly 100 (FIG. 3) with the distal end of outer tube 32.

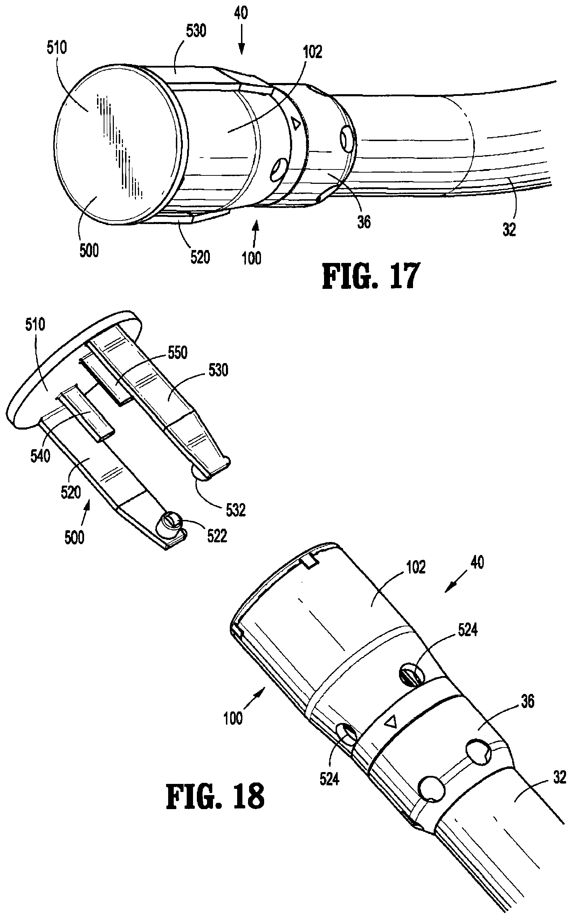

Referring to FIGS. 17-21, distal head portion 40 of surgical stapling apparatus 10 (FIG. 1) includes anvil assembly 400 (FIG. 1), described above, that is releasably engagable with the distal end of approximation assembly 200 (FIG. 3), and a replaceable stapling assembly 100 that is releasably engagable with the distal end of elongated central body portion 30 (FIG. 3). A safety cap 500 (FIGS. 17-18) is also provided for engagement about the distal end of replaceable stapling assembly 100 when not in use, e.g., during shipping and storage. Replaceable stapling assembly 100 (or portions thereof) is configured as a disposable component that is to be replaced with a new replaceable stapling assembly 100 (or portions thereof) after each firing. The remaining components of surgical stapling apparatus 10 (FIG. 1) are configured as reusable, sterilizable components, although one or more of these components may alternatively be configured as a disposable component. Other configurations are also contemplated. Distal head portion 40 will be described in greater detail below.

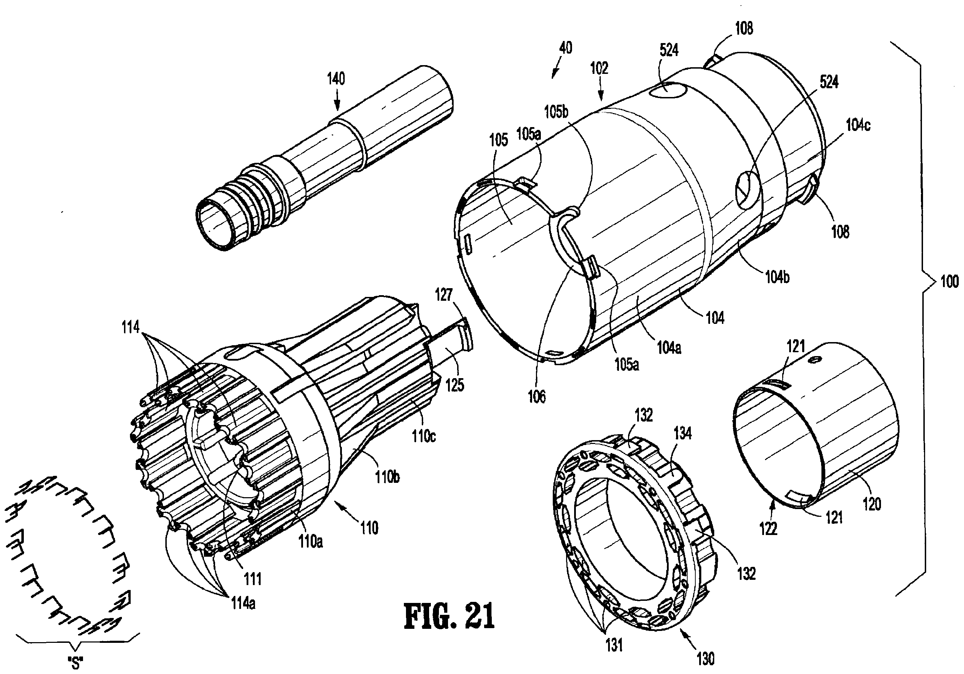

Replaceable stapling assembly 100 will now be described in detail with respect to FIGS. 17-21. Referring initially to FIGS. 17-18, and as mentioned above, a safety cap 500 is provided for engagement about the distal end of replaceable stapling assembly 100 when not in use, e.g., during shipping and storage. Safety cap 500 includes a disc member 510 configured for positioning about the distal end of stapling assembly 100, a pair of outer arms 520, 530 extending proximally from disc member 510, and a pair of inner posts 540, 550 extending proximally from disc member 510. Each outer arm 520, 530 includes an inwardly-extending protrusion 522, 532 disposed at its free end. Protrusions 522, 532 are configured for receipt within apertures 524 defined within outer housing portion 104 of shell assembly 102 of stapling assembly 100 to retain safety cap 500 about the distal end of stapling assembly 100. Arms 520, 530 may be formed from a resiliently flexible material so as to bias protrusions 522, 532 into apertures 524, although other engagement mechanisms for releasably retaining safety cap 500 about the distal end of stapling assembly 100 are also contemplated. Inner posts 540, 550 are configured for insertion into stapling assembly 100 to help retain safety cap 500 in position about the distal end of stapling assembly 100 and to inhibit distal movement of pusher back 110 (FIG. 21) as will be described below to prevent the inadvertent ejection of staples "S" (FIG. 21) from stapling assembly 100 during shipping or the like.

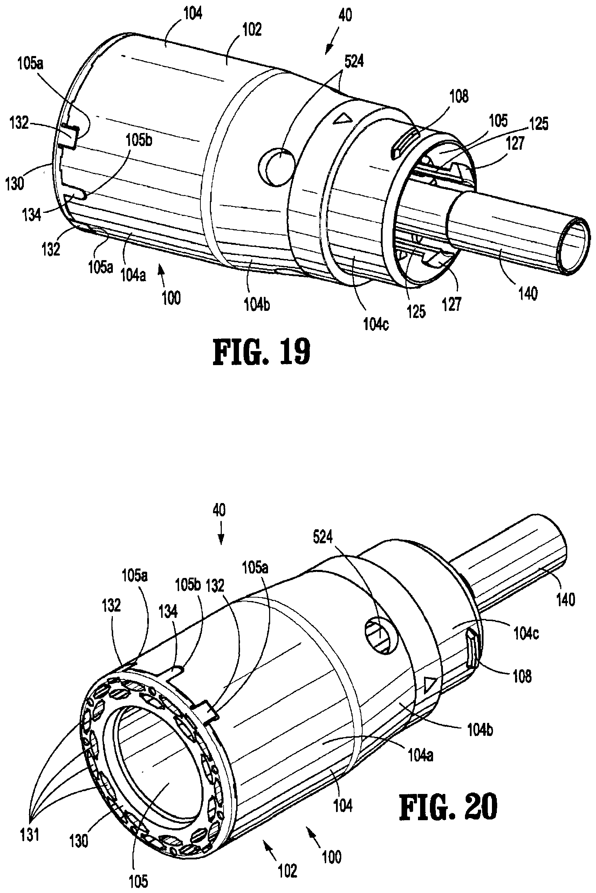

Referring to FIGS. 19-21, replaceable stapling assembly 100 includes a shell assembly 102, a pusher back 110, a cylindrical knife 120, and a staple guide cap 130. Shell assembly 102 includes an outer housing portion 104 and an inner guide portion 106. Outer housing portion 104 defines a throughbore 105 and includes a distal cylindrical section 104a, a central conical section 104b, and a proximal cylindrical section 104c. Distal cylindrical section 104a includes a slot 105a and a plurality of recesses 105b. Slot 105a is configured to receive a protrusion 132 formed on staple guide cap 130 to properly align staple guide cap 130 with pusher back 110. Recesses 105b are configured for engagement with tabs 134 formed on staple guide 130 for securing staple guide cap 130 to staple back 110.

Proximal cylindrical section 104c of outer housing portion 104 of shell assembly 102 includes a pair of tabs 108 formed an inner surface thereof. Tabs 108 are configured to selectively engage threads 38 (FIGS. 15-16) formed on the inner surface of distal bushing 36 (FIGS. 15-16) to releasably theradably engage shell assembly 102 and outer tube 32 (FIG. 15) with one another. In this manner, shell assembly 102 of stapling assembly 100 may be removed from stapling apparatus 10 (FIG. 1) subsequent to use and stapling apparatus 10 (FIG. 1) may be reloaded with another stapling assembly 100 and reused.

Pusher back 110 includes a central throughbore 111 which is slidably positioned about inner guide portion 106 of shell 102. Pusher back 110 includes a distal cylindrical section 110a which is slidably positioned within distal cylindrical section 104c of outer housing portion 104, a central conical section 110b, and a proximal smaller diameter cylindrical section 110c. Pusher back 110 further includes a pair of proximally-extending arm members 125. Arm members 125 each include a finger 127 that is configured for insertion into and locking engagement within annular recess 128a of collar 128 disposed at distal end 350b of pusher end tube 350 of pusher link 320 (see FIG. 11). Thus, with pusher link 320 (FIG. 11) engaged with pusher back 110, actuation of firing trigger 24 (FIG. 11) urges pusher back 110 distally through outer housing portion 104 to eject staples "S" from stapling assembly 100.

With particular reference to FIG. 21, distal cylindrical section 110a of pusher back 110 includes a plurality of distally extending fingers 114 dimensioned to be slidably received within slots 131 formed in staple guide cap 130 to eject staples "S" therefrom. Distal ends 114a of fingers 114 define a groove for engaging staples "S." Cylindrical knife 120 is retained within central throughbore 111 of pusher back 110 by a pair of tabs 121. Alternately, knife 120 may be retained within pusher back 110 using adhesives, crimping, pins, etc. A distal end of knife 120 includes a circular cutting edge 122. A rigid bushing 140 is supported in the proximal end of inner guide portion 106 of shell 102. Bushing 140 defines a throughbore dimensioned to slidably receive anvil retainer 240 (FIG. 5) and center rod assembly 420 of anvil assembly 400 (FIG. 1).

In operation, when pusher linkage 320 (FIG. 10) is advanced distally in response to actuation of trigger 24 (FIG. 10), pusher back 110 is advanced distally within outer housing portion 104 of shell assembly 102. Advancement of pusher back 110 advances fingers 114 through slots 131 of staple guide cap 130 to advance staples "S" positioned within slots 131 and eject staples "S" from staple guide cap 130 into staple deforming pockets (not shown) formed on an opposed surface of anvil head assembly 410 of anvil assembly 400 (FIG. 1). Since knife 120 is secured to pusher back 110, knife 120 is also advanced distally to core tissue.

The use of surgical stapling apparatus 10, disassembly of surgical stapling apparatus 10 for sterilization of the reusable components and replacement of the disposable components, and reassembly of surgical stapling apparatus 10 for subsequent use is now described. Adjustment of tissue gap adjustment mechanism 260 (FIG. 9A) is also described and may be effected during manufacturing, assembly, between uses, or at any other suitable point where setting and/or changing the minimum tissue gap is desired.

With general reference to FIGS. 1 and 22, in use, safety cap 500 (FIGS. 17-18) is initially removed from the distal end of stapling assembly 100. Next, distal head portion 40 of surgical stapling apparatus 10 inserted into an internal surgical site, before or after engagement of distal head portion 40 with the anvil assembly 400. Next, anvil assembly 400 and stapling assembly 100 are positioned adjacent tissue to be stapled. At this point, anvil assembly 400 is in an unapproximated position and screw 220 of approximation assembly 200 (FIGS. 5-9) is in its distal-most position. This position of anvil assembly 400 may be visually confirmed by viewing indicator 276 of indicator bar 270 (FIG. 3) through window 25 (FIG. 4) formed in stationary handle 22. As shown in FIG. 2, trigger lock 360 is disposed in the locked position at this point such that actuation of firing trigger 24 is inhibited. Trigger 24 is further prevented from being actuated by engagement of protrusion 306 (FIGS. 10-11) of trigger 24 with screw 220 (FIGS. 5-9), as detailed above.

Once distal head portion 40 of surgical stapling apparatus 10 is positioned as desired, anvil assembly 400 may be approximated relative to stapling assembly 100 to clamp tissue therebetween via manipulating approximation knob 26. Tissue can be secured between anvil assembly 400 and stapling assembly 100 using conventional techniques such as using purse-string sutures, resilient bands, or the like. Knob 26 may be rotated to approximate anvil assembly 400 relative to stapling assembly 100 to clamp tissue therebetween until the minimum tissue gap between anvil assembly 400 and stapling assembly 100, which is set via tissue gap adjustment mechanism 260 (FIG. 9A), is achieved. Movement of the anvil assembly 400 to the approximated position can be visually confirmed once visual indicator 278 (FIG. 3) is viewable through window 25 (FIG. 4).

With anvil assembly 400 disposed in the approximated position and tissue clamped between anvil head 410 and staple guide cap 130 of stapling assembly 100, firing assembly 300 (FIGS. 10-11) may be actuated to staple and core the clamped tissue. In order to allow for firing, trigger lock 360 is rotated from the locked position to the unlocked position. In the approximated position of anvil assembly 400, recess 225 formed in screw 220 of approximation assembly 200 (see FIGS. 5-9) is aligned with protrusion 306 (FIGS. 10-11) formed on trigger 24 to permit actuation of trigger 24, provided trigger lock 260 is disposed in the unlocked position.

With trigger lock 360 and protrusion 306 (FIGS. 10-11) no longer inhibiting actuation of trigger 24, surgical stapling apparatus 10 may be actuated. In order to fire stapling apparatus 10, trigger 24 is compressed towards stationary handle 22, which urges pusher link assembly 320 (FIGS. 10-11) distally through outer tube 32 to urge pusher back 110 (FIG. 21). With additional reference to FIG. 21, distal translation of pusher back 110 relative to staple guide cap 130 urges pusher back 110 to engage and eject staples "S" from staple guide cap 130, through tissue, and into anvil head 410 of anvil assembly 400, which form staples "S" about tissue. Knife 120 is moved concurrently with pusher back 110 such that knife 120 is likewise advanced distally to core tissue.

Continuing with general reference to FIGS. 1 and 22, in one exemplary method of use, surgical stapling apparatus 10 is used to perform a circular anastomosis. Typically, circular anastomoses are required during procedures for removing a portion of a diseased vessel such as the colon or the intestine. During such a procedure, the diseased portion of the vessel is removed and the end portions of the remaining first and second vessel sections are joined together using the surgical stapling apparatus 10.

During such a procedure using the surgical stapling apparatus 10, prior to removing the diseased vessel portion from the diseased vessel, anvil assembly 400 with a removable trocar (not shown) attached thereto is positioned in the first vessel section on a first side of the diseased portion. A removable trocar which is suitable for use with anvil assembly 400 is disclosed in the Gresham '444 patent. After the diseased vessel portion is removed and the open ends of the first and second vessel sections have been sutured, the distal end of apparatus 10 is positioned in the second vessel section on the other side of the diseased vessel portion which has been removed. At this time, the removable trocar is pushed through the suture line in the end of the first vessel section and removed from the anvil assembly. Next, trocar tip 247 of anvil retainer 240 is pushed through the suture line in the second vessel section and is joined to the center rod of the anvil assembly 400. The surgical stapling apparatus 10 can now be approximated and fired in the manner discussed above to join the ends of the first and second vessel sections and core out any tissue obstructing the vessel lumen.

At the completion of the stapling operation, surgical stapling apparatus 10 may be removed from the internal surgical site. More specifically, anvil assembly 400 may be configured to pivot to a low-profile configuration after firing and upon un-approximation of anvil assembly 400 relative to stapling assembly 100 to facilitate removal of surgical stapling apparatus 10 from the internal surgical site. A suitable tilting mechanism is described in the Milliman '187 patent or the Gresham '444 patent. Alternatively, anvil assembly 400 need not have a pivotal head and may be removed from the surgical site in the same orientation as it was advanced into the surgical site.

Upon removal from the internal surgical site at the completion of the surgical procedure (or prior to use), surgical stapling apparatus 10 may be disassembled to facilitate sterilization of the reusable components and replacement of the disposable components. Adjustment of tissue gap adjustment mechanism 260 (FIG. 9A) may also be effected at this time.

Referring still to FIGS. 1 and 22, to disassemble surgical stapling apparatus 10, anvil assembly 400 is first removed from anvil retainer 240 by moving anvil assembly 400 to the unapproximated position and separating anvil assembly 400 from anvil retainer 240 using sufficient force to disengage center rod assembly 420 from annular protrusion 248 (FIG. 5). Anvil assembly 400 is configured as a sterilizable, reusable component although it is also contemplated that anvil assembly be configured as a reusable component.

Once anvil assembly 400 has been removed, stapling assembly 100 may be disengaged from surgical stapling apparatus 10. More specifically, stapling assembly 100 is disengaged from the distal end of outer tube 32 by rotating shell assembly 102 relative to outer tube 32 to disengage tabs 108 (FIGS. 20-21) from threads 38 (FIG. 16) of distal bushing 36. Thereafter, shell assembly 102 is squeezed inwardly and translated distally to disengage fingers 127 (FIGS. 20-21) of arms 125 (FIGS. 20-21) from collar 128 of distal pusher end 350 (FIG. 11) to fully disengage stapling assembly 100 from outer tube 32. Once disengaged, stapling assembly 100 may then be removed from positioning about anvil retainer 240 and may be disposed of, although it is also contemplated that one or more components of stapling assembly 100 be sterilizable for reuse.

Referring to FIGS. 3D-3F, in order to disassemble stationary handle 22 in preparation for sterilization, proximal bushing 34 is disengaged from the distal ends of handle sections 22a, 22b by rotating proximal bushing 34 relative to stationary handle 22, and collar 27 is disengaged from the proximal ends of handle sections 22a, 22b by rotating collar 27 relative to stationary handle 22. Once proximal bushing 34 and collar 27 have been disengaged from the proximal and distal ends of stationary handle 22, handle sections 22a, 22b may be moved relative to one another from the closed position (FIG. 3D) to the open position (FIG. 3F).

In order to move handle sections 22a, 22b from the closed position (FIG. 3D) to the open position (FIG. 3F), handle sections 22a, 22b are initially moved apart from one another and relative to discs 50, 51 (see FIG. 3E with respect to disc 50). In particular, handle sections 22a, 22b are translated apart from one another and relative to discs 50, 51 such that pins 56a, 57a are translated from the inner ends of slots 56, 57 (FIGS. 3B and 3C) to the outer ends of slots 56, 57, respectively. This outward translation of handle sections 22a, 22b provides clearance between handle sections 22a, 22b and the internal components retained within stationary handle 22. More specifically, the outward translation of handle sections 22a, 22b withdraws pivot member 316 from slots 31 (FIG. 2) and withdraws the wings of proximal end 330a of elongated pusher tube 330 from the channels defined within handle sections 22a, 22b (FIG. 3).

Once sufficient clearance has been achieved between handle sections 22a, 22b and the internal components of handle portion 20, the free sides of handle sections 22a, 22b, i.e., the sides of handle sections 22a, 22b opposite the pin-slot engagement of handle sections 22a, 22b and discs 50, 51, are rotated apart from one another about pins 56a, 57a relative to support discs 50, 51 to the open position. In the open position of handle sections 22a, 22b, the proximal components of approximation assembly 200 and firing assembly 300 are exposed, facilitating adjustment and/or removal of any or all of these components, as detailed below.

With momentary reference to FIG. 9A, at this point, if it is desired to change the minimum tissue gap setting, set screw 262 is loosened, and asymmetrical washer 264 is rotated about set screw 262 and relative to screw stop 250 to the desired position. Once the desired position is achieved, set screw 262 may be re-tightened to maintain screw stop 250 in the desired position on screw 220, thus defining the desired minimum tissue gap.

Referring to FIGS. 1, 2, and 22, once handle sections 22a, 22b have been pivoted to the open position (FIG. 3F), approximation assembly 200 and firing assembly 300 may be removed from stationary handle 22. Thus, with stationary handle 22 opened, and with approximation assembly 200 and firing assembly 300 removed from handle portion, sterilization of each of these components for reuse may be readily achieved. Alternatively, one or more of theses components may be configured as a disposable component and, thus, may be replaced with a new component rather than being sterilized. Additionally or alternatively, sterilization may be effected with stationary handle 22 in the open position (FIG. 3F) but without the need to remove approximation assembly 200 and/or firing assembly 300.

Once the reusable components, e.g., handle sections 22a, 22b, approximation assembly 200, and firing assembly 300, have been sterilized and the replaceable components, e.g., stapling assembly 100, replaced, surgical stapling apparatus 10 may be reassembled for subsequent use in reverse order of disassembly. As can be appreciated, the above-described cycle of use, disassembly, sterilization and replacement, adjustment, and reassembly, may be repeated for a plurality of usage cycles.

## Claims

1. A surgical stapling apparatus (10), comprising:
a handle portion (20) defining a proximal end and a distal end;
a body (30) extending distally from the handle portion (20);
a stapling assembly (100) supported on a distal end of the body (30);
an anvil assembly (400);
a drive screw (220) supported within the handle portion (20) and operably coupled to the anvil assembly (400), the drive screw (220) defining a threaded aperture (228) and being movable relative to the stapling assembly (100) to move the anvil assembly (400) relative to the stapling assembly (100) between a spaced-apart position and an approximated position; and
a tissue gap adjustment mechanism (260) disposed within the handle portion (20) and including:
a stop member (250) supported on the drive screw (220), the stop member (250) including a transverse slot (254), first and second flanges (255a, 255b) extending proximally and distally of the transverse slot (254), the stop member (250) configured to abut a stop surface within the handle portion (20) to prevent further proximal movement of the drive screw (220) within the handle portion (20) and set a minimum tissue gap between the anvil assembly (400) and the stapling assembly (100);
an asymmetrical polygonal washer (264) defining an eccentrically positioned aperture (268) and including a plurality of pairs of opposed flat sides (269a-269h), the washer (264) being dimensioned to be positioned between the first and second flanges such that each of the pairs of opposed flat sides (269a-269h) can be selectively positioned between and in engagement with the first and second flanges (255a, 255b); and
a set screw (262) configured for insertion through the aperture (268) of the washer (264) and the transverse slot (254), and into the threaded aperture (228) to fix the stop member (250) relative to the drive screw (220),
wherein the washer (264) is repositionable about the set screw (262) to position a selected pair of the opposed flat sides (269a-269h) between and in engagement with the first and second flanges (255a, 255b), at least two of the pairs of opposed flat sides (269a-269h) when engaged with the first and second flanges (255a, 255b) being spaced to position the aperture (268) of the washer (264) at different longitudinal locations in relation to the transverse slot such that the longitudinal position of the stop member (250) in relation to the drive screw (220) can be selectively varied by positioning a different pair of opposed flat sides (269a-269h) in engagement with the first and second flanges (255a, 255b) to selectively change the minimum tissue gap.

2. The surgical stapling apparatus (10) according to claim 1, wherein the washer (264) defines an octagonal configuration; and/or wherein the washer (264) is configured and dimensioned such that the minimum tissue gap is adjustable between 4.55mm and 5.45mm.

3. The surgical stapling apparatus (10) according to claim 1 or claim 2, wherein the washer (264) is configured and dimensioned such that the minimum tissue gap is incrementally adjustable at a step size of 0.15mm between 4.55mm and 5.45mm.

4. The surgical stapling apparatus (10) according to any preceding claim, further comprising at least one indicator (256) disposed on the washer (264) and an indicator (258) disposed on at least one of the flanges (255a, 255b) for indicating a selected setting of the tissue gap adjustment mechanism (260).

5. The surgical stapling apparatus (10) according to any preceding claim, wherein the set screw (262) is configured to be loosened to facilitate repositioning of the washer (264) about the set screw (262) and is configured to be tightened to fix the position of the washer (264) and the stop member (250) on the drive screw (220).

6. The surgical stapling apparatus (10) according to any preceding claim, further comprising an approximation knob (26) extending from the handle portion (20), the approximation knob (26) coupled to the drive screw (220) and selectively actuatable to move the anvil assembly (400) between the spaced-apart position and the approximated position.

7. The surgical stapling apparatus (10) according to claim 6, wherein the drive screw (220) defines a helical channel (221) and the approximation knob (26) is coupled to a ball (218) disposed within the helical channel (221) such that rotation of the approximation knob (26) effects translation of the drive screw (220).

8. The surgical stapling apparatus (10) according to any preceding claim, further comprising a firing assembly (300) including a trigger (24) coupled to the handle (20) and a firing link (310) coupled to the stapling assembly (100), the firing link (310) configured for distal translation through the body in response to actuation of the trigger (24) to eject a plurality of surgical staples (S) from the stapling assembly (100).

9. The surgical stapling apparatus (10) according to any preceding claim, wherein the handle (20) is formed from first and second handle sections (22a, 22b), the first and second handle sections (22a, 22b) being releasably engagable with one another.

## Patentansprüche

1. Chirurgische Klammervorrichtung (10), umfassend:
einen Griffabschnitt (20), der ein proximales Ende und ein distales Ende definiert;
einen Körper (30), der sich von dem Griffabschnitt (20) distal erstreckt;
eine Klammeranordnung (100), die an einem distalen Ende des Körpers (30) gestützt wird;
eine Ambossanordnung (400);
eine Antriebsschraube (220), die innerhalb des Griffabschnitts (20) gestützt wird und mit der Ambossanordnung (400) wirkgekoppelt ist, wobei die Antriebsschraube (220) eine Gewindeöffnung (228) definiert und relativ zu der Klammeranordnung (100) beweglich ist, um die Ambossanordnung (400) relativ zu der Klammeranordnung (100) zwischen einer beabstandeten Position und einer angenäherten Position zu bewegen; und
einen Gewebespaltanpassungsmechanismus (260), der innerhalb des Griffabschnitts (20) eingerichtet ist und einschließt:
ein Anschlagelement (250), das auf der Antriebsschraube (220) gestützt wird, wobei das Anschlagelement (250) einen Querschlitz (254),einen ersten und einen zweiten Flansch (255a, 255b), die sich von dem Querschlitz (254) proximal und distal erstrecken, einschließt, wobei das Anschlagelement (250) konfiguriert ist, um an eine Anschlagoberfläche innerhalb des Griffabschnitts (20) zu stoßen, um eine weitere proximale Bewegung der Antriebsschraube (220) innerhalb des Griffabschnitts (20) zu verhindern und einen minimalen Gewebespalt zwischen der Ambossanordnung (400) und der Klammeranordnung (100) einzustellen;
eine asymmetrische polygonale Unterlegscheibe (264), die eine exzentrisch positionierte Öffnung (268) definiert und eine Vielzahl von Paaren gegenüberliegender flacher Seiten (269a-269h) einschließt, wobei die Unterlegscheibe (264) so dimensioniert ist, dass sie zwischen dem ersten und dem zweiten Flansch positioniert ist, so dass jedes der Paare gegenüberliegender flacher Seiten (269a-269h) zwischen und in Eingriff mit dem ersten und dem zweiten Flansch (255a, 255b) wahlweise positioniert werden kann; und
eine Einstellschraube (262), die für eine Einsetzung durch die Öffnung (268) der Unterlegscheibe (264) und den Querschlitz (254) und in die Gewindeöffnung (228) konfiguriert ist, um das Anschlagelement (250) relativ zu der Antriebsschraube (220) zu fixieren,
wobei die Unterlegscheibe (264) um die Einstellschraube (262) herum neupositionierbar ist, um ein ausgewähltes Paar der gegenüberliegenden flachen Seiten (269a-269h) zwischen und in Eingriff mit dem ersten und dem zweiten Flansch (255a, 255b) zu positionieren, wobei mindestens zwei der Paare gegenüberliegender flacher Seiten (269a-269h), wenn sie mit dem ersten und dem zweiten Flansch (255a, 255b) in Eingriff stehen, beabstandet sind, um die Öffnung (268) der Unterlegscheibe (264) an verschiedenen Längspositionen in Bezug auf den Querschlitz zu positionieren, so dass die Längsposition des Anschlagelements (250) in Bezug auf die Antriebsschraube (220) wahlweise variiert werden kann, durch Positionieren eines verschiedenen Paares gegenüberliegender flacher Seiten (269a-269h) in Eingriff mit dem ersten und dem zweiten Flansch (255a, 255b), um den minimalen Gewebespalt wahlweise zu ändern.

2. Chirurgische Klammervorrichtung (10) nach Anspruch 1, wobei die Unterlegscheibe (264) eine achteckige Konfiguration definiert; und/oder wobei die Unterlegscheibe (264) konfiguriert und dimensioniert ist, so dass der minimale Gewebespalt zwischen 4,55 mm und 5,45 mm anpassbar ist.

3. Chirurgische Klammervorrichtung (10) nach Anspruch 1 oder 2, wobei die Unterlegscheibe (264) konfiguriert und dimensioniert ist, so dass der minimale Gewebespalt schrittweise bei einer Schrittgröße von 0,15 mm zwischen 4,55 mm und 5,45 mm anpassbar ist.

4. Chirurgische Klammervorrichtung (10) nach einem der vorstehenden Ansprüche, ferner umfassend mindestens einen Anzeiger (256), der auf der Unterlegscheibe (264) eingerichtet ist, und einen Anzeiger (258), der auf mindestens einem der Flansche (255a, 255b) eingerichtet ist, zum Anzeigen einer ausgewählten Einstellung des Gewebespaltanpassungsmechanismus (260).

5. Chirurgische Klammervorrichtung (10) nach einem der vorstehenden Ansprüche, wobei die Einstellschraube (262) konfiguriert ist, um gelöst zu werden, um das Neupositionieren der Unterlegscheibe (264) um die Einstellschraube (262) zu erleichtern, und konfiguriert ist, um angezogen zu werden, um die Position der Unterlegscheibe (264) und des Anschlagelements (250) an der Antriebsschraube (220) zu fixieren.

6. Chirurgische Klammervorrichtung (10) nach einem der vorstehenden Ansprüche, ferner umfassend einen Näherungsknopf (26), der sich von dem Griffabschnitt (20) erstreckt, wobei der Näherungsknopf (26) mit der Antriebsschraube (220) gekoppelt und wahlweise betätigbar ist, um die Ambossanordnung (400) zwischen der beabstandeten Position und der angenäherten Position zu bewegen.

7. Chirurgische Klammervorrichtung (10) nach Anspruch 6, wobei die Antriebsschraube (220) einen spiralförmigen Kanal (221) definiert und der Näherungsknopf (26) mit einer Kugel (218) gekoppelt ist, die innerhalb des spiralförmigen Kanals (221) eingerichtet ist, so dass eine Drehung des Näherungsknopfes (26) eine Translation der Antriebsschraube (220) bewirkt.

8. Chirurgische Klammervorrichtung (10) nach einem der vorstehenden Ansprüche, ferner umfassend eine Schussanordnung (300), die einen Auslöser (24) einschließt, der mit dem Griff (20) gekoppelt ist, und eine Schussverbindung (310), die mit der Klammeranordnung (100) gekoppelt ist, wobei die Schussverbindung (310) für die distale Translation durch den Körper als Reaktion auf die Betätigung des Auslösers (24) konfiguriert ist, um eine Vielzahl von chirurgischen Klammern (S) aus der Klammeranordnung (100) auszustoßen.

9. Chirurgische Klammervorrichtung (10) nach einem der vorstehenden Ansprüche, wobei der Griff (20) aus einem ersten und einem zweiten Griffbereich (22a, 22b) gebildet ist, wobei der erste und der zweite Griffbereich (22a, 22b) lösbar miteinander in Eingriff bringbar sind.

## Revendications

1. Appareil d'agrafage chirurgical (10), comprenant :
une partie poignée (20) définissant une extrémité proximale et une extrémité distale ;
un corps (30) s'étendant de manière distale de la partie poignée (20) ;
un ensemble d'agrafage (100) supporté sur une extrémité distale du corps (30) ;
un ensemble enclume (400) ;
une vis d'entraînement (220) supportée à l'intérieur de la partie poignée (20) et couplée fonctionnellement à l'ensemble enclume (400), la vis d'entraînement (220) définissant une ouverture filetée (228) et étant mobile par rapport à l'ensemble d'agrafage (100) pour déplacer l'ensemble enclume (400) par rapport à l'ensemble d'agrafage (100) entre une position espacée et une position rapprochée ; et
un mécanisme d'ajustement d'espace de tissu (260) disposé à l'intérieur de la partie poignée (20) et comportant :
un élément de butée (250) supporté sur la vis d'entraînement (220), l'élément de butée (250) comportant une fente transversale (254), des première et seconde brides (255a, 255b) s'étendant de manière proximale et distale de la fente transversale (254), l'élément de butée (250) étant configuré pour venir en butée contre une surface de butée à l'intérieur de la partie poignée (20) pour empêcher un mouvement proximal supplémentaire de la vis d'entraînement (220) à l'intérieur de la partie poignée (20) et définir un espace de tissu minimal entre l'ensemble enclume (400) et l'ensemble d'agrafage (100) ;
une rondelle polygonale asymétrique (264) définissant une ouverture positionnée de manière excentrique (268) et comportant une pluralité de paires de côtés plats opposés (269a-269h), la rondelle (264) étant dimensionnée pour être positionnée entre les première et seconde brides de telle sorte que chacune des paires de côtés plats opposés (269a-269h) peut être positionnée sélectivement entre les première et seconde brides (255a, 255b) et en prise avec les première et seconde brides (255a, 255b) ; et
une vis de calage (262) conçue pour être insérée à travers l'ouverture (268) de la rondelle (264) et la fente transversale (254), et dans l'ouverture filetée (228) pour fixer l'élément de butée (250) par rapport à la vis d'entraînement (220),
dans lequel la rondelle (264) est repositionnable autour de la vis de calage (262) pour positionner une paire sélectionnée des côtés plats opposés (269a-269h) entre les première et seconde brides (255a, 255b) et en prise avec les première et seconde brides (255a, 255b), au moins deux des paires de côtés plats opposés (269a-269h) lorsqu'elles sont en prise avec les première et seconde brides (255a, 255b) étant espacées pour positionner l'ouverture (268) de la rondelle (264) à différents emplacements longitudinaux par rapport à la fente transversale de telle sorte que la position longitudinale de l'élément de butée (250) par rapport à la vis d'entraînement (220) peut être amenée à varier sélectivement en positionnant une paire différente de côtés plats opposés (269a-269h) en prise avec les première et seconde brides (255a, 255b) pour modifier sélectivement l'espace de tissu minimal.

2. Appareil d'agrafage chirurgical (10) selon la revendication 1, dans lequel la rondelle (264) définit une configuration octogonale ; et/ou dans lequel la rondelle (264) est conçue et dimensionnée de telle sorte que l'espace de tissu minimal est ajustable entre 4,55 mm et 5,45 mm.

3. Appareil d'agrafage chirurgical (10) selon la revendication 1 ou la revendication 2, dans lequel la rondelle (264) est conçue et dimensionnée de telle sorte que l'espace de tissu minimal est ajustable de manière incrémentielle à une taille de pas de 0,15 mm entre 4,55 mm et 5,45 mm.

4. Appareil d'agrafage chirurgical (10) selon une quelconque revendication précédente, comprenant en outre au moins un indicateur (256) disposé sur la rondelle (264) et un indicateur (258) disposé sur au moins l'une des brides (255a, 255b) permettant d'indiquer un réglage sélectionné du mécanisme d'ajustement d'espace de tissu (260).

5. Appareil d'agrafage chirurgical (10) selon une quelconque revendication précédente, dans lequel la vis de calage (262) est conçue pour être desserrée pour faciliter le repositionnement de la rondelle (264) autour de la vis de calage (262) et est conçue pour être serrée pour fixer la position de la rondelle (264) et de l'élément de butée (250) sur la vis d'entraînement (220).

6. Appareil d'agrafage chirurgical (10) selon une quelconque revendication précédente, comprenant en outre un bouton d'approximation (26) s'étendant à partir de la partie poignée (20), le bouton d'approximation (26) étant accouplé à la vis d'entraînement (220) et pouvant être actionné de manière sélective pour déplacer l'ensemble enclume (400) entre la position espacée et la position rapprochée.

7. Appareil d'agrafage chirurgical (10) selon la revendication 6, dans lequel la vis d'entraînement (220) définit un canal hélicoïdal (221) et le bouton d'approximation (26) est accouplé à une bille (218) disposée à l'intérieur du canal hélicoïdal (221) de telle sorte que la rotation du bouton d'approximation (26) entraîne une translation de la vis d'entraînement (220).

8. Appareil d'agrafage chirurgical (10) selon une quelconque revendication précédente, comprenant en outre un ensemble de déclenchement (300) comportant une gâchette (24) accouplée à la poignée (20) et une liaison de déclenchement (310) accouplée à l'ensemble d'agrafage (100), la liaison de déclenchement (310) étant conçue pour une translation distale à travers le corps en réponse à l'actionnement de la gâchette (24) pour éjecter une pluralité d'agrafes chirurgicales (S) de l'ensemble d'agrafage (100).

9. Appareil d'agrafage chirurgical (10) selon une quelconque revendication précédente, dans lequel la poignée (20) est formée à partir de première et seconde sections de poignée (22a, 22b), les première et seconde sections de poignée (22a, 22b) pouvant venir en prise de manière amovible l'une avec l'autre.
